# EUROPEAN PATENT APPLICATION

(11) **EP 2 201 970 A1**
(43) Date of publication of application: **30.06.2010**
(21) Application number: 08022327.4
(22) Date of filing: 23.12.2008
(51) Int. Cl.: A61M 5/24, A61M 5/32

(54) **Drug delivery device**

(71) Applicant: Sanofi-Aventis Deutschland GmbH, 65929 Frankfurt am Main (DE)
(72) Inventor: The designation of the inventor has not yet been filed

(57) **Abstract**

The present invention relates to a drug delivery (5) comprising a housing (10) with a proximal end (28) and a distal end (22), a cartridge holder (14) adapted to retain a cartridge, the cartridge holder (14) being secured to the housing (10) and having an at least partially transparent side wall, and a cap (40) being capable of covering the distal end (22) of the drug delivery device, the cap (40) being rotationally free movable with respect to the housing (10) and comprising a window aperture (44), the window aperture (44) being capable of displaying information revealed by the cartridge holder (14). Furthermore, using a cap (40) for covering the distal end (22) of the drug delivery device (5) is disclosed.

## Description

The present invention generally relates to drug delivery devices. Furthermore, the present invention relates to using a cap for covering a distal end of a drug delivery device.

Drug delivery devices are generally known in the art for the administration of a medicinal product, for example insulin, growth hormones or other drugs, in particular medicinal products being suitable for self-administration by a patient.

Some drug delivery devices are configured to deliver a plurality of doses. One particular example of such a drug delivery device is described in the document EP 1 923 084 A1. There, a drug delivery device is shown where a user may activate the drug delivery device. The drug delivery device includes a drive mechanism suitable for use in pen-type injectors, wherein a number of pre-set doses of medicinal product can be administered. A needle unit can be attached to the drug delivery device for dispensing the medicinal product into a patient's skin. After usage of the drug delivery device, the distal end of the device can be covered by a cap.

Additionally, some drug delivery devices are configured to allow setting of different dose sizes which are to be delivered.

It is generally advisable that the drug delivery device is covered by a cap after usage so as to prevent contact with the needle and/or contamination of the device.
In US 2008/0269688 A1, a dose indicating assembly in a pharmaceutical injection device is shown. The dose indicating assembly includes an external housing barrel extending in an axial direction, and a dial at least partially disposed within the housing barrel. The dial is screwably movable in the axial direction relative to the barrel during dose setting. The dial includes an outer radial periphery with a plurality of parallel arrays of dose indicia provided thereon, each of the plurality of arrays of dose indicia provided in a helical pattern on the periphery. The dose indicating assembly also includes means for viewing the dose indicia of a selectively chosen one of the plurality of arrays.

In US 6,491,665, a syringe assembly is shown having a syringe body adapted to be filled with an injectable liquid and having an annular neck defining an axis and having an axially outwardly open outlet. The neck is adapted to carry a needle. An elastomeric plug engaged axially inward with the neck closes the outlet and a holding ring is fixed to the neck below the plug. A retaining sleeve around the neck is fixed to the holding ring and a cup-shaped safety cap is fixed to the plug. The safety cap is formed with at least one radially through going aperture that gives the end user the ability to see if the plug is properly in place before cracking off the cap.

A further injection device with a scale being located on a housing is known from the document DE 298 18 721 U1.

It is an aim of the present invention to provide for an improved drug delivery device. In particular, a drug delivery device should be provided, which allows for an efficient information retrieval by a user before, during or after operation of the device.

For this aim, a drug delivery device comprises a housing with a proximal end and a distal end, a cartridge holder adapted to retain a cartridge, the cartridge holder being secured to the housing and having an at least partially transparent side wall, and a cap being capable of covering the distal end of the drug delivery device, the cap being rotationally free movable with respect to the housing and comprising a window aperture, the window aperture being capable of displaying information revealed by the cartridge holder.

In such a drug delivery device, both the cartridge holder and the cap are fixed with respect to the housing. The cartridge holder is covered by the cap on the distal end of the drug delivery device. After engagement of the cap, information revealed by the cartridge holder can be displayed through the window aperture.

Furthermore, the cap, the cartridge holder, and the cartridge can be constructed such that the information revealed by the cartridge holder can be imaged through the window aperture, so as to allow a user to quickly gain information regarding the drug delivery device. Consequently, the user can receive information regarding the fill status, the type of medicinal product or the like, even without removing the cap from the drug delivery device.

This is in particular useful when a user requires treatment with different medicinal products. According to an aspect of the invention, a set of drug delivery devices can easily be disentangled from each other by the user without removing the cap from some or all of the devices.

In a first embodiment, the drug delivery device comprises a piston rod, the piston rod being adapted to drive a piston so as to engage the piston into the cartridge, the position of the piston within the cartridge being visible through the window aperture.

As the drug is dispensed from the drug delivery device, the piston progressively moves forward towards the distal end of the cartridge. Through the at least partially transparent side wall the position of the piston is visible for the user. Accordingly, the filling status of the drug delivery device can be easily recognized by viewing the piston through the window aperture.

This is in particular useful when the user needs to work through a course of medication that involves a set of disposable drug delivery devices. According to this embodiment, filling status information for each member of the set of disposable drug delivery devices can easily be retrieved through the window aperture.

In a further embodiment, the window aperture displays information attached to the cartridge holder.

According to this embodiment, the user can view information which is provided on the cartridge holder. This can include the type of the medical product contained in the cartridge, information being associated with the prescription of the medicinal product or the like. Displaying information through the window aperture is in particular useful when employing the drug delivery device as an expendable product, where usually only one specific medicinal product is dispensed.

In a further embodiment, the window aperture displays information transmitted from the cartridge through the at least partially transparent side wall of the cartridge holder.

According to this embodiment, information directly attached to the cartridge can be displayed through the window aperture. Usually, cartridges can be labeled according to their content. Consequently, the risk of a potential misuse of the drug delivery device is significantly reduced especially when the patient is required to apply different medicinal products. As the fill status and other type of information is permanently displayed, even when the cap is attached to the housing, operation of the medication delivery device is facilitated as the user can gather relevant information at all time without opening the device. Hence, time consuming selection among different devices is no longer necessary, for example.

Furthermore, the window aperture provides a framing of the displayed information as only these parts of the cartridge holder underneath the window aperture are visible for the user. Accordingly, the information is presented in a clearer, i.e. less visually confusing way.

In one embodiment, the displayed information includes a dosage scale.

According to this embodiment, the user is provided with key information regarding the filling status of the medication delivery device. This information is in particular useful when the user needs to work through a course of medication that involves a number of disposable drug delivery devices. There, different medication delivery devices are usually contained within a kit. The user may wish to store used and unused devices of the kit within the same compartment, e.g. a box or the like. According to this embodiment, the selection of the correct device is facilitated as filling status information can easily be retrieved through the dosage scale. In combination with the advancing piston driven by the piston rod, the filling status information provided by the position of the piston can even be transferred into a number of doses remaining in the cartridge.

In a further embodiment, the dosage scale is printed or moulded onto the cartridge holder.

According to this example, the dosage scale can be provided as printed symbols which can be designed for good visibility, e.g. by providing a respective colour which enables a good contrast with respect to the cartridge holder. The dosage scale can be provided already during manufacturing of the cartridge holder, e.g. by printing or by injection moulding. This reduces the number of necessary manufacturing steps and thus may lead to a reduction in manufacturing costs.

In a further embodiment, the dosage scale is provided at least partially rotationally symmetric on the cartridge holder with respect to a longitudinal axis between the proximal end and the distal end.

In this example, the dosage scale is visible through the window aperture under all or almost all different orientations of the cap with respect to the cartridge holder. The dosage scale can be line shaped elements being provided as annular circles on the cartridge holder, as circular segments or as ticks. As such, the user can view the dosage scale irrespective of the actual position of the cap on the cartridge holder.

In a further embodiment, at least one symbol is printed onto the cap so as to provide further information relating to the dosage scale.

According to this embodiment, attention of the user is attracted as the dosage information can be related to further information, e.g. imminent emptiness of the cartridge contained in the drug delivery device. Hence, operability of the drug delivery device is enhanced.

In a further embodiment, at least one symbol is moulded onto the cap so as to provide further information relating to the dosage scale.

In this example, further information can be provided already during manufacturing of the cartridge holder, e.g. by injection moulding. This reduces the number of necessary manufacturing steps and thus may lead to a reduction in manufacturing costs.

In a further embodiment, a plurality of symbols is present, the symbols comprising numbers.

In this example, the filling status of the drug delivery device can be is displayed by the advancing piston which progressively moves underneath the window aperture. Accordingly, the position of the piston within the cartridge is visible through the partially transparent cartridge holder. Furthermore, information regarding the filling status, i.e. about the number of doses remaining to be administered from the device, is presented to the user in a clear and less confusing manner.

In a further embodiment, the window aperture comprises a plurality of sub-windows.

By sub-dividing the window aperture into a plurality of sub-windows; the information being displayed is framed so as to guide the user in an attempt to retrieve information from the drug delivery device. Accordingly, unnecessary or confusing information which might be present on the cartridge or the cartridge holder can be suppressed by selecting the location of the sub-windows in an appropriate way. Consequently, the framing provided by the sub-windows of the window aperture causes a pre-selection of information to be displayed and the information is presented in a clearer, i.e. less visually confusing way to the user.

In a further embodiment, the sub-windows indicate dosage information as a progressive scale.

In this example, the information regarding the filling status of the drug delivery device is displayed by the advancing piston which progressively moves underneath the corresponding sub-windows of the window aperture. Accordingly, the position of the piston within the cartridge is visible through the partially transparent cartridge holder. Furthermore, the information regarding the filling status is presented in a familiar format to the user which is, for example, similar as a fuel gauge or a battery charge level indicator. Information about the number of doses remaining to be administered from the device can be differentiated from other information on the device and is thus presented in a clear and less confusing manner.

In a further embodiment, the cap is fabricated from an injection moulded plastic.

According to this embodiment, the design of the cap is simplified and conventional techniques can be employed for producing the cap. For example, the cap can be provided as a single injection moulded piece fabricated from thermoplastic materials, e.g. polystyrene, polyamide, polyethylene, or the like.

According to a further embodiment, the cap comprises a retaining member, the retaining member being capable of a rotationally free movable engagement into a corresponding mating retaining member on the housing.

In this example, the cap can be fixed with respect to the housing. Hence, information revealed by the cartridge holder can be displayed through the window aperture. It is however, not necessary to provide a rotationally fixing of the cap with respect to the housing. Furthermore, the user can engage the cap in arbitrary positions relative to the housing. Hence, no false orientation with respect to the housing is possible and closing of the device by the cap is facilitated.

For the above mentioned aim, a cap is used for covering the distal end of the drug delivery device with a housing with a proximal end and a distal end and with a cartridge holder adapted to retain a cartridge, the cartridge holder being secured to the housing, wherein the cap comprises a window aperture, the window aperture being capable of displaying information revealed by the cartridge holder.

Other features will become apparent from the following detailed description when considered in conjunction with the accompanying drawings.

In the drawings:
Figure 1 schematically shows a simplified perspective side view of a drug delivery device according to an embodiment;
Figure 2 schematically shows a simplified side view of a part of a drug delivery device according to an embodiment;
Figure 3 schematically shows a simplified side view of a part of a drug delivery device according to an embodiment; and
Figures 4A to 4D schematically show a simplified side view of a part of a drug delivery device according to an embodiment.

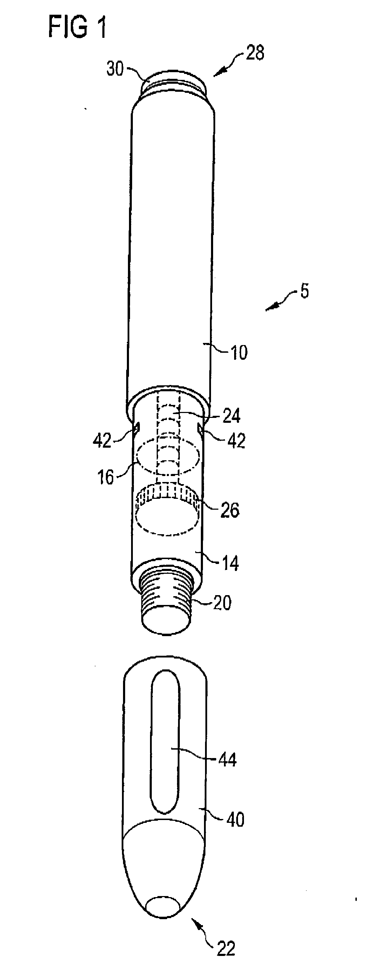

In Figure 1, an embodiment of a drug delivery device 5 is shown which can be an injector for a liquid medication. The drug delivery device may be configured to deliver a plurality of fixed or user-settable doses of a drug. The drug delivery device 5 may be a pen-type device. The drug delivery device 5 comprises a housing 10 which can be formed from a single or from multiple pieces.

In the embodiment shown in Figure 1, the housing 10 is attached to a cartridge holder 14, wherein a cartridge 16 containing a medical product or drug can be located. The cartridge holder 14 may be secured against movement with respect to the housing 10. The proximal end of the cartridge 16 is indicated by a dashed line in Figure 1.

A needle unit (not shown in Figure 1) can be located at the distal end 22 of the drug delivery device 5. Through the needle unit the medical product can be injected into a patient. The needle unit can be secured to a needle holder 20 by a threaded engagement. Usually, the needle holder 20 forms a part of the cartridge holder 14.

Delivery of the medical product can be performed by means of a piston rod 24, which can be moved into the distal direction with respect to the cartridge 16. A piston 26 which is retained in the cartridge 16 and seals the cartridge on the proximal side 28 may be moved in the distal direction 22 with respect to the cartridge by the piston rod 24. The cartridge holder 14 is fabricated from a transparent or a translucent material, so as to allow viewing of the position of the piston 26 within the cartridge 16.

It should be noted that the description of the drug delivery device 5 as shown in Figure 1 is merely illustrative. Other elements might be necessary in order to achieve full functionality. For example, a dispense button 30 and a drive mechanism (not shown in Figure 1) can be present, which are configured to apply the adjusted dose value and move the piston rod and the piston 24 in the distal direction such that the adjusted amount of the medicinal product is dispensed upon pressing the dispense button 30.

In order to prevent contamination of the drug delivery device 5, a cap 40 is attached to the drug delivery device 5, when the drug delivery device 5 is not in use. The cap 40 covers the distal end 22 of the drug delivery device 5 including the needle holder 20 and the cartridge holder 14.

The cap 40 and the cartridge holder 14 can comprise a set of retaining members which are capable of securing the cap 40 with respect to the cartridge holder 14 so as to be rotationally free movable with respect to a longitudinal axis being defined between the distal end 22 and the proximal end 28 of the drug delivery device 5. The retaining member (not shown in Figure 1) located on the inner surface of the cap 40 is capable of engaging into a corresponding mating retaining member 42 on the cartridge holder 14.

As shown in Figure 1, the mating retaining member 42 comprises a clamp capable of engaging into a corresponding recess on the inner surface of the cap 40. The recess of the cap can be formed as a groove, wherein the clamp can engage so as to be fixed to the cartridge holder 14. Along the groove, the cap 40 is rotationally free movable with respect to the cartridge holder 14.

However, other configurations including, for example, a recess on the cap 40 and a corresponding clamp on the cartridge holder 14 are conceivable as well. The mating retaining member 42 can be an annular groove, for example. The clamp of the cap 40 can engage into the annular groove, so as to be fixed to the cartridge holder 14, Again, the cap 40 is rotationally free movable with respect to the cartridge holder 14.

As shown in Figure 1, the cap 40 includes a window aperture 44. Through the window aperture 44 information revealed by the cartridge holder 14 can be displayed. Information revealed by the cartridge holder 14 can be displayed through the window aperture 44.

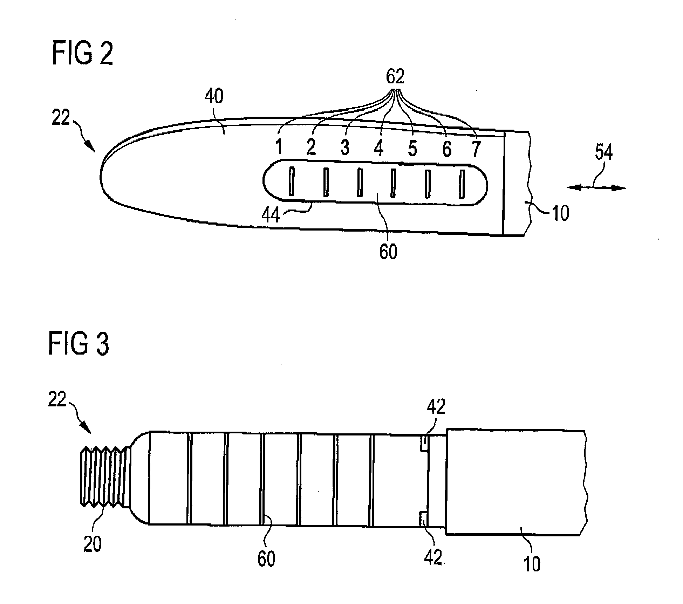

Making now reference to Figure 2, the cap 40 and the distal end of drug delivery device 5 are shown in more detail. In the embodiment shown in Figure 2, the cap 40 and the cartridge holder 14 are constructed such that the information revealed by the cartridge holder includes a dosage scale 60, which can be imaged through the window aperture 44.

The window aperture 44 is formed in the embodiment shown in Figure 2 as an elongated slit. However, other forms for the window aperture are conceivable as well, for example rectangular or any other suitable configuration. Furthermore, the cap 40 can also include a further aperture window (not shown in Figure 2) for displaying further information.

In the embodiment shown in Figure 2, the cap 40 includes several symbols 62, which are printed or moulded as numbers on the outer surface of the cap 40. The numbers can indicate the fill status of the cartridge 16 in the cartridge holder 14. In Figure 2, numbers ranging from one to seven are shown. This design is in particular useful for fixed dosage pen-type injectors, where no user controlled dosage is applied and therefore, the movement of the piston is directly related to the number of dosages left.

Making now reference to Figure 3, the cartridge holder 14 on the distal part of drug delivery device 5 is shown in more detail.
In the embodiment shown in Figure 3, the cartridge holder 14 contains the dosage scale 60. Further information regarding the type of the medicinal product or related to the production of the medical product, e.g. a production date or bar coded production information, can also be present (not shown in Figure 3). It should be noted that part of the information can also be printed on the cartridge 16 and is then transmitted through the transparent side walls of the cartridge holder 14.

The dosage scale 60 is provided rotationally symmetric on the cartridge holder 14 with respect to a longitudinal axis 54 between the proximal end 28 and the distal end 22. The dosage scale 60 is visible through the window aperture 44 under different orientations of the cap 40 with respect to the cartridge holder 14. The dosage scale 60 is formed as a line shaped element as an annular circle on the cartridge holder 14, for example.

As can been seen from Figure 2 and Figure 3, the window aperture 44 provides a framing of the displayed information as only these parts of the cartridge holder underneath the window aperture 44 are visible. In the embodiment of Figure 2 and Figure 3 only the dosage scale is visible through the window aperture 44 and a user can retrieve fill status information in a clear way without being confused by the further information symbols.

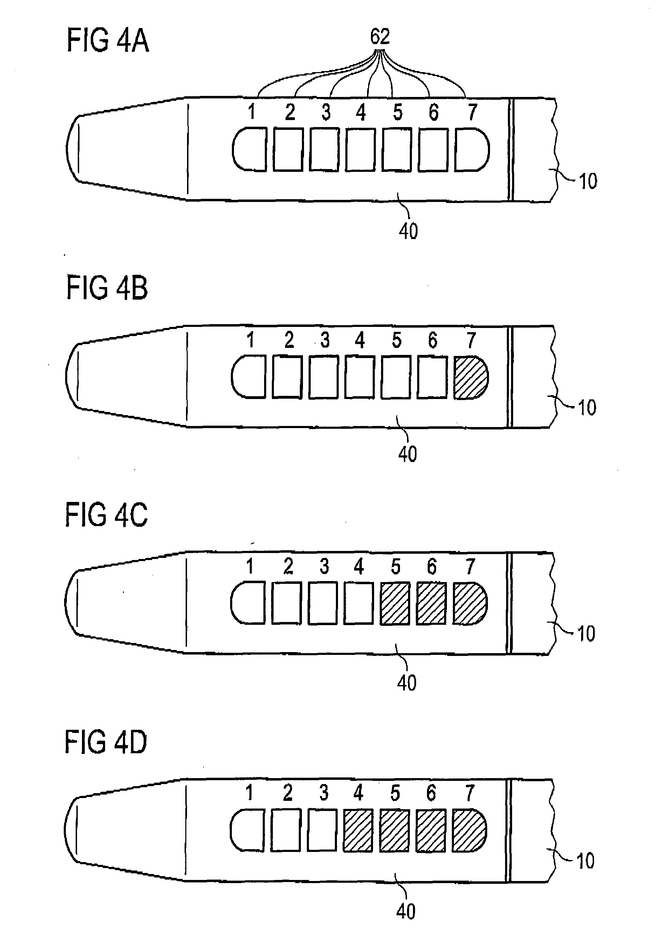

It should be noted that the invention is not restricted to provide only fill status information. Furthermore the cap 40 can contain further symbols which provide additional information for the user. For example, letters or numbers can be printed or molded onto the cap 40 (not shown in Figure 2), Making now reference to Figures 4A to 4D, a further embodiment of the drug delivery device 5 is shown,

In Figures 4A to 4D, the position of the piston within the cartridge 16 is visible through the window aperture 44. As the drug is dispensed from the drug delivery device 5, the piston 26 progressively moves forward towards the distal end 22 of the cartridge 16. Through the at least partially transparent side wall the position of the piston 26 is visible and the filling status of the drug delivery device 5 can be detected by viewing the piston 26 through the window aperture 44.

In combination with the dosage scale 60, the filling status information provided by the position of the piston 26 can be transferred into a number of doses remaining in the cartridge, which is in particular useful for fixed dosage devices.

As shown in Figure 4A to 4D, the window aperture comprises a plurality of sub-windows 70. The sub-windows 70 indicate dosage information as a progressive scale. The information regarding the filling status is presented as symbols 60 ranging from one to seven. Information about the number of doses remaining to be administered from the device can be easily retrieved.

The embodiment of Figure 4A corresponds to a new device with no dosage dispensed so far. Figure 4B shows the same device with one dosage applied, i.e. with six dosages remaining.
Figure 4C shows the device after three applied and Figure 4D after four applied dosages.

Other implementations are within the scope of the following claims. Elements of different implementations may be combined to form implementations not specifically described herein.

### Reference Numerals

- Drug delivery device: 5
- Housing: 10
- Cartridge holder: 14
- Cartridge: 16
- Needle holder: 20
- Distal end: 22
- Piston rod: 24
- Piston: 26
- Proximal end: 28
- Dispense button: 30
- Cap: 40
- Mating retaining member: 42
- Window aperture: 44
- Longitudinal axis: 54
- Dosage scale: 60
- Symbols: 62
- Sub-window: 70

## Claims

1. A drug delivery device comprising:
a housing (10) with a proximal end (28) and a distal end (22), a cartridge holder (14) adapted to retain a cartridge, the cartridge holder (14) being secured to the housing (10) and
having an at least partially transparent side wall, and
a cap (40) being capable of covering the distal end (22) of the drug delivery device, the cap (40) being rotationally free movable with respect to the housing (10) and comprising a window aperture (44), the window aperture (44) being capable of displaying information revealed by the cartridge holder (14) .

2. The drug delivery device according to claim 1, comprising a piston rod (24), the piston rod (24) being adapted to drive a piston (26) so as to engage the piston (26) into the cartridge (16), the position of the piston (26) within the cartridge (16) being visible through the window aperture (44).

3. The drug delivery device according to claim 1 or 2, wherein the window aperture (44) displays information attached to the cartridge holder (14).

4. The drug delivery device to any of claims 1 to 3, wherein the window aperture (44) displays information transmitted from the cartridge (16) through the at least partially transparent side wall of the cartridge holder (14).

5. The drug delivery device according to any of claims 1 to 4, wherein the displayed information includes a dosage scale (60).

6. The drug delivery device according to any of claims 1 to 5, wherein the dosage scale (60) is printed or moulded onto the cartridge holder (14).

7. The drug delivery device according to claim 5, wherein the dosage scale is provided rotionally symmetric onto the cartridge holder (14) with respect to a longitudinal axis (54) between the distal end (22) and the proximal end (28).

8. The drug delivery device according to any of claims 5 to 7, wherein at least one symbol is printed onto the cap (40) so as to provide further information relating to the dosage scale.

9. The drug delivery device according to any of claims 5 to 7, wherein at least one symbol is moulded onto the cap (40) so as to provide further information relating to the dosage scale.

10. The drug delivery device according to any of claims 5 to 7, wherein a plurality of symbols is present, the symbols comprising numbers.

11. The drug delivery device according to any of claims 1 to 10, wherein the window aperture (44) comprises a plurality of sub-windows (70).

12. The drug delivery device according to claim 10 and 11, wherein the sub-windows (70) indicate dosage information as a progressive moving scale.

13. The drug delivery device according to any of claims 1 to 12, wherein the cap (40) is fabricated from an injection moulded plastic.

14. The drug delivery device according to any of claims 1 to 13, wherein the cap (40) comprises a retaining member, the retaining member being capable of a rotationally free movable engagement into a corresponding mating retaining member on the housing.

15. Using a cap for covering the distal end (22) of the drug delivery device (5) with a housing (10) with a proximal end (28) and a distal end (22) and with a cartridge holder (14) adapted to retain a cartridge (16), the cartridge holder (14) being secured to the housing (10), wherein the cap (40) comprises a window aperture (44), the window aperture (44) being capable of displaying information revealed by the cartridge holder (14).
